# DEMANDE DE BREVET EUROPEEN

(11) **EP 3 815 650 A1**
(43) Date de publication de la demande: **05.05.2021**
(21) Numéro de dépôt: 20306321.9
(22) Date de dépôt: 04.11.2020
(51) Int. Cl.: A61F 2/80, A61F 2/78, A61F 2/50, A61F 2/54

(54) **PROCEDE DE FABRICATION PAR IMPRESSION 3D D'UN DISPOSITIF D'EMBOITURE POUR APPAREILLAGE ORTHOPEDIQUE DE TYPE PROTHESE**

(30) Priorité: 04.11.2019 FR 1912353; 16.12.2019 FR 1914540
(71) Demandeur: MED IN 3D, 59650 Villeneuve d'Ascq (FR)
(72) Inventeur: BOUCHER, François, 59000 Lille (FR)
(74) Mandataire: Cabinet Rifflart Vandenbossche

(57) **Abrégé**

L'invention concerne un procédé de fabrication d'une emboîture (100) pour prothèse d'un membre inférieur ou supérieur d'un patient amputé, ledit procédé comprenant:
- une acquisition (S0) d'une représentation tridimensionnelle d'un moignon du membre inférieur ou supérieur dudit patient amputé ;
- une génération (S1) d'un modèle numérique d'emboîture (100) à partir de ladite représentation tridimensionnelle, ledit modèle numérique comprenant une enveloppe surfacique représentative d'un corps principal creux ouvert en partie supérieure et comprenant une paroi latérale et un fond formant un chausson dimensionné pour recevoir au moins partiellement ledit moignon ;
- une détermination (S2) d'une position d'une zone de réception se présentant sous la forme d'une empreinte préformée dans le corps principal pour recevoir des moyens d'assujettissement ;
- une mise à jour (S3) dudit modèle par intégration de ladite zone ;
- une impression 3D (S4) dudit corps principal comprenant ladite empreinte préformée en fonction dudit modèle.

## Description

### Domaine technique

La présente invention concerne le domaine des appareillages orthopédiques de type prothèse pour membre inférieur ou supérieur, et plus particulièrement les dispositifs d'emboiture pour prothèse fémorale ou tibiale.

L'objet de la présente invention porte sur un procédé fabrication d'un dispositif d'emboîture simple à mettre en œuvre permettant à un orthoprothésiste de réaliser un dispositif d'emboîture confortable et adapté à la morphologie du patient en un minimum de temps, notamment après amputation.

### Art antérieur

Une prothèse dans le cadre de la présente invention peut être considérée comme un dispositif prothétique visant à appareiller un patient amputé ou présentant une malformation nécessitant une prothétisation.

Dans le cadre de la présente invention, on s'intéresse principalement aux prothèses fémorales et tibiales ; c'est-à-dire les prothèses destinées aux patients amputés au-dessus du genou (prothèse fémorale ou trans-fémorale) ou en dessous du genou (prothèse tibiale ou trans-tibiale). On remarque cependant que la présente invention peut également s'appliquer au domaine des prothèses pour membres supérieurs.

L'objectif des prothèses fémorales et tibiales est de permettre au patient de retrouver une certaine autonomie à travers un gain de mobilité : la marche.

Pour atteindre cet objectif, une prothèse fémorale ou tibiale comprend classiquement les éléments suivants :
- un dispositif d'emboîture (ou emboîture) destiné à recevoir le moignon ;
- éventuellement un manchon destiné à envelopper le moignon du patient pour faciliter la mise en place du moignon dans l'emboîture ;
- des moyens d'assujettissement (de type plongeur ou moyens de dépressurisation) pour assujettir ledit moignon à l'emboîture ;
- des pièces de jonction prothétiques ;
- un pied prothétique et éventuellement un genou prothétique.

Le dispositif d'emboîture, qui est destiné à recevoir le moignon soit directement soit par l'intermédiaire du manchon, assure principalement trois fonctions dont la suspension de la prothèse, l'appui de celle-ci sur le moignon et l'activation de la prothèse par le moignon lors de la marche prothétique.

La qualité de conception du dispositif d'emboîture et sa réalisation s'avèrent essentielles pour assurer ces fonctions : le dispositif d'emboîture doit être confortable pour le patient et facile à chausser ; il doit également présenter une bonne tenue mécanique sur le moignon afin notamment de ne pas perdre le moignon lors de la phase oscillante de la marche.

Lors de la prise en charge d'un patient amputée, on distingue la prothèse dite définitive de la prothèse provisoire.

La prothèse provisoire comprend un dispositif d'emboîture dont la durée de vie est limitée (une semaine à quelques mois) ; il s'agit du premier dispositif d'emboîture que reçoit le patient après amputation une fois que son état médical permet un appareillage. Ce dispositif d'emboîture est le plus souvent réalisé *in situ* dans le centre de rééducation du patient par un orthoprothésiste.

Le patient peut en fonction de l'évolution de son état de santé recevoir plusieurs dispositifs d'emboîture provisoires pendant son parcours de rééducation : le moignon du patient amputé est en effet instable et inflammatoire après amputation. Celui-ci va ensuite se stabiliser (changement de forme et de volume principalement). Il peut donc être nécessaire pendant le parcours de rééducation du patient de fabriquer plusieurs dispositifs d'emboîture afin de s'adapter à la forme et au volume du moignon.

Cette emboîture provisoire est réalisée dans un matériau plastique transparent du type PolyEthylène Téréphtalate Glycol ou PETG.

Ensuite, le patient se fait appareiller avec une prothèse définitive. Une telle prothèse comprend un dispositif d'emboîture qui est réalisé cette fois dans un matériau composite (fibres de verre ou fibres de carbone + résine acrylique ou époxy) ; un tel dispositif est fabriqué par un orthoprothésiste privé une fois l'état médical du patient stabilisé et sa rééducation terminée.

Il est généralement prévu de réitérer la fabrication de l'emboîture environ tous les deux ans au minimum.

On sait que le process de fabrication d'une emboîture provisoire reste relativement artisanal et nécessite le savoir-faire d'un orthoprothésiste. De façon classique, ce process comprend principalement les quatre étapes suivantes :
- une prise d'empreinte (obtention du négatif d'un membre résiduel) et réalisation du positif ;
- une rectification du positif obtenu ;
- un formage de l'emboîture ; et
- un montage de la prothèse.

Ces quatre étapes définissent la séquence générique du processus de fabrication des emboîtures.

En fonction des techniques et des matériaux employés, l'état de l'art permet une multitude de séquences de réalisation.

La séquence dite de référence correspond à la méthode enseignée et majoritairement utilisée pour la fabrication d'emboîtures. Elle se définit par la succession des étapes suivantes :
- un moulage plâtré ;
- un coulage du positif ;
- une rectification du plâtre ;
- un thermoformage par emboutissage ;
- un usinage de l'emboîture et une fixation de la pièce sous-jacente.

L'étape de moulage permet une obtention du négatif du membre résiduel (appelé également le moignon). Le négatif est obtenu par un enroulement de bandes plâtrées sur le membre résiduel du patient, préalablement protégé des éclaboussures et de la poussière occasionnée par du film transparent étirable. Un temps de séchage et de maintien du membre résiduel dans une position précise est nécessaire.

Le négatif est ensuite retiré du membre résiduel après séchage ; le patient est alors libéré des éléments protecteurs puis nettoyé au besoin. L'orthoprothésiste nettoie ensuite la salle et jette les reliquats de consommables utilisés.

Cette étape nécessite donc des bandes plâtrées, un point d'eau et une bassine. Cette étape est généralement réalisée dans une salle de moulage dédiée.

Le moulage est une étape salissante ; un film transparent étirable et/ou une alèse sont souvent requis aussi bien pour la protection de la salle que pour le patient.

Cette étape de moulage reste donc contraignante, voire éprouvante physiquement, pour le patient et son confort.

L'étape de coulage du positif permet l'obtention d'un positif du membre résiduel à partir du moulage. L'orthoprothésiste amène en salle de plâtre le négatif obtenu lors du moulage et le rehausse à l'aide de bande plâtrées, afin d'y couler une préparation de plâtre en poudre et d'eau pour obtenir après séchage un positif (correspondant à un double en plâtre du membre résiduel du patient). Il est ensuite nécessaire de retirer du positif le négatif. L'orthoprothésiste peut alors nettoyer la zone et jeter le négatif et la rehausse.

L'ensemble est laissé de côté le temps du séchage (ou étape de durcissement).

Cette étape est réalisée dans une salle de plâtre dédiée et nécessite comme précédemment un point d'eau avec une bassine, des bandes plâtrées et du plâtre en poudre.

Encore une fois, cette étape reste technique et complexe et requiert du personnel qualifié, un temps de séchage et des consommables.

Suite à l'obtention du positif, l'orthoprothésiste doit rectifier celui-ci en salle de plâtre à l'aide d'un matériel adapté (spatule, râpe, outil de ponçage et de lissage, etc.).

Cette étape de rectification consiste plus précisément en la modification du positif pour obtenir la meilleure congruence moignon-emboîture. Elle est réalisée dans la salle de plâtre. Lors de cette étape, l'orthoprothésiste repère à l'aide d'un crayon les zones à modifier (zone à creuser ou à recharger) sur le positif.

Ensuite, à l'aide de différentes râpes (ronde, semi ronde et/ou plate), il réalise l'ensemble des modifications nécessaires. Régulièrement l'orthoprothésiste vérifie sa rectification en relevant des mesures : hauteurs, circonférences et diamètres à l'aide d'un pied à coulisse et d'un mètre ruban. Une fois terminée, la forme est lissée à l'aide d'un grillage et d'un lé de plâtre (fait à partir d'un mélange plâtre en poudre et eau).

Avant de commencer l'étape de fabrication, le plâtre doit sécher.

On envoie ensuite en atelier.

Il faut préparer le plâtre pour la fabrication :
- positionner le plâtre à l'envers sur un socle réalisant un vide d'air ;
- recouvrir le plâtre d'un lycra et de magnésie pour favoriser la circulation de l'air et faciliter le démoulage.

Il faut ensuite préparer la plaque pour le thermoformage :
- positionner une plaque de thermoplastique (souvent PETG) sur un cadre support
- déposer la plaque dans un four préchauffé à environ 170°C

Une fois suffisamment chauffée, elle est thermoformée par emboutissage sur le positif rectifié.

Le vide d'air est mis en place au fur et à mesure que l'orthoprothésiste accompagne la matière sur le pourtour du positif.

Un temps de refroidissement est alors nécessaire avant la découpe et l'usinage de la future emboîture.

Dans le cas où l'emboîture nécessite un accrochage distal à plongeur à inclure dans le plastique, un gabarit est positionné par l'orthoprothésiste à l'endroit opportun (extrémité de l'emboîture). Il faut alors faire attention à ne pas le déplacer lors du thermoformage.

Ensuite, on usine l'emboîture avec la fixation et l'instrumentation des différents éléments d'accroché : ancre (ou platine de fixation), etc.

L'élément prothétique sous-jacent à l'emboîture requiert bien souvent une ancre de fixation ; celle-ci est fixé avec de la colle et une bande de résine.

Cette étape n'est pas systématique. Une pièce de jonction peut alternativement être prise dans le thermoformage pour permettre de fixer directement les éléments prothétiques sous-jacents. L'orthoprothésiste doit enfin découper et poncer l'emboîture avant de finaliser le montage avec les pièces prothétiques sous-jacentes.

Il se rend pour ce faire dans la salle des machines.

Mais avant cela, il doit séparer l'emboîture du plâtre : il utilise alors un marteau et un burin afin de casser et de jeter le positif.

L'enseignement pratique de cette séquence décrite ci-dessus est très important pour les orthoprothésistes. Elle permet d'acquérir l'expertise nécessaire à l'exercice du métier d'orthoprothésiste et constitue une base de connaissances partagée et essentielle aux bonnes pratiques.

Ce savoir-faire est le résultat d'un entraînement répété dans lequel sont réalisées de multiples manipulations dont notamment des manipulations directes sur le patient lors de l'étape de moulage plâtré.

Cet examen clinique permet aux orthoprothésistes de s'instruire sur toutes les caractéristiques du moignon en rapport avec l'appareillage :
- une mobilisation du membre résiduel pour une appréciation des amplitudes et des limitations articulaires ;
- une mobilisation des masses molles pour une évaluation du serrage à appliquer dans la zone de tolérance ;
- un repérage des zones possiblement conflictuelles (osseuses, douloureuses, cicatricielles, faible sensibilité, etc.).

Cette séquence de référence renvoie donc à un procédé défini et appliqué depuis plus de trente ans.

Le Demandeur soumet toutefois que cette séquence de référence présente de nombreux inconvénients au regard des outils technologiques actuels et des délais de réalisation.

Par ailleurs, cette séquence de référence demeure très contraignante aussi bien pour l'orthoprothésiste que le patient : une exposition prolongée au plâtre (potentiellement dérangeante et salissante) et une position imposée à maintenir pour le patient pendant la durée de prise de moulage et de séchage.

On connaît dans l'état de la technique les documents qui ont tenté d'apporter des solutions aux inconvénients ci-dessus.

Ainsi, les documents US2018/0243111 et US2018/0243112 proposent l'utilisation de l'impression 3D pour concevoir un dispositif d'emboîture.

On connaît également le document WO 2017/151577 qui propose une technique d'impression 3D pour la réalisation d'une emboîture.

En revanche, le confort du patient avec un dispositif d'emboîture tel que proposé dans ces documents n'est pas pleinement satisfaisant : le dispositif proposé est adapté uniquement au système d'accroché avec plongeur et le moignon du patient reste instable dans l'emboîture.

### Objet et résumé de la présente invention

La présente invention vise à améliorer la situation décrite ci-dessus.

La présente invention vise notamment à remédier à au moins un des différents problèmes techniques mentionnés ci-dessus en proposant un dispositif d'emboîture simple de fabrication venant s'adapter à tous les systèmes d'accroché et permettant un assemblage solidaire avec le moignon pour stabiliser fermement le moignon dans l'emboîture.

A cet effet, l'objet de la présente invention concerne selon un premier aspect un dispositif d'emboîture pour prothèse d'un membre inférieur ou supérieur d'un patient amputé.

Selon la présente invention, le dispositif comporte un corps principal creux ouvert en partie supérieure.

De préférence, le corps principal comprend ici une paroi latérale et un fond formant ensemble un chausson dimensionné pour recevoir au moins partiellement un moignon du membre inférieur ou supérieur du patient amputé.

Avantageusement, le dispositif selon la présente invention comporte des moyens d'assujettissement du dispositif d'emboîture au moignon.

De préférence, ces moyens d'assujettissement sont actionnables manuellement par actionnement d'un actionneur.

Avantageusement, le corps principal comprend une zone de réception préformée ménagée dans le fond du chausson.

Avantageusement, le corps principal comprend en outre une gouttière préformée traversant la paroi latérale en partie inférieure du corps principal pour déboucher dans la zone de réception. Avantageusement, une portion proximale de l'actionneur est fixée solidairement dans la gouttière et une portion distale de l'actionneur reste accessible au patient lorsque le moignon est en position dans le chausson.

Ainsi, grâce à cet agencement, caractéristique de la présente invention, et notamment grâce à à la conception de ce corps principal intégrant une gouttière préformée débouchant sur une zone de réception préformée, il devient possible de mettre à disposition des orthoprothésistes et des patients amputés un dispositif d'emboîture assurant un bon confort du patient grâce à un assujettissement du dispositif avec le moignon en facilitant les opérations de façonnage et de fabrication.

De préférence, le corps principal se présente sous la forme d'une pièce monobloc.

De préférence, le corps principal est réalisé par impression 3D à partir d'une modélisation numérique du moignon.

Dans un mode de réalisation avantageux, les moyens d'assujettissement comprennent un système de dépressurisation dont l'actionnement assujettit le dispositif d'emboîture au moignon par dépressurisation.

Dans un autre mode de réalisation avantageux qui peut être combiné avec le précédent mode, les moyens d'assujettissement sont configurés pour assujettir le dispositif d'emboîture au moignon par un assujettissement mécanique d'un plongeur fixé sur un manchon enveloppant le moignon, ledit plongeur se présentant sous la forme d'une tige rigide prolongeant axialement le moignon lorsque le manchon enveloppe le moignon.

Selon une variante, les moyens d'assujettissement comprennent une pièce d'assemblage logée dans la zone de réception. Une telle pièce logée dans le fond du chausson permet d'assembler solidairement entre eux le corps principal et le moignon.

De préférence, la zone de réception présente une forme complémentaire à la pièce d'assemblage de manière à ce que la pièce d'assemblage vienne s'emboîter dans la zone de réception.

De préférence, la pièce d'assemblage se présente sous forme d'un cylindre ayant un épaulement sur son pourtour périphérique.

D'autres formes pourront être envisagées dans le cadre de la présente invention pour favoriser cet emboîtement.

Avantageusement, la pièce d'assemblage comprend un canal radial dont une extrémité débouche sur la paroi latérale externe de la pièce d'assemblage et dont l'autre extrémité débouche sur un canal axial traversant axialement la pièce d'assemblage.

De préférence, une portion du plongeur est introduite dans le canal axial lors du positionnement du moignon dans le chausson.

Avantageusement, le corps principal comprend un orifice de passage ménagé dans le fond dut chausson.

De préférence, cet orifice de passage est aligné avec le canal axial de la pièce d'assemblage de manière à ce que, lorsque le moignon du patient est positionné dans le chausson, une portion du plongeur traverse le canal axial et l'orifice de passage pour déboucher à l'extérieur du corps principal.

Avantageusement, la pièce d'assemblage comprend un système de verrouillage/déverrouillage logé dans le canal radial.

De préférence, le système de verrouillage/déverrouillage comprend une pièce de verrouillage/déverrouillage mobile montée sur ressort.

De préférence, une telle pièce est apte à présenter deux positions dont une position de déverrouillage dans laquelle la pièce reste dans le volume défini par le canal radial et une position de verrouillage dans laquelle une portion de la pièce s'engage dans le volume défini par le canal axial de manière à venir en butée contre le plongeur pour maintenir en position celui-ci dans le canal axial.

De préférence, le passage de la position de verrouillage à la position de déverrouillage est enclenché par l'actionnement de l'actionneur. Cet enclenchement se fait par exemple par l'actionnement de la portion distale de l'actionneur (une molette ou un bouton poussoir par exemple).

Dans un mode de réalisation avantageux, le dispositif selon la présente invention comprend un détrompeur mis en œuvre entre la zone de réception et la pièce d'assemblage.

De préférence, un tel détrompeur est configuré pour garantir un positionnement et une orientation unique de la pièce d'assemblage lorsque celle-ci est positionnée dans la zone de réception de manière à aligner la gouttière et le canal radial tout en bloquant en rotation la pièce d'assemblage dans la zone de réception.

Selon une variante, le détrompeur comporte :
- une empreinte mécanique femelle ou mâle ménagée dans la zone de réception, et
- une empreinte mécanique mâle ou femelle ménagée dans la pièce d'assemblage,
lesdites empreintes mécaniques mâle et femelle présentant chacune une géométrie complémentaire l'une de l'autre.

Selon une autre variante, le détrompeur comporte sur la zone de réception et/ou la pièce d'assemblage un ou plusieurs aimants présentant une polarité alternée, l'alignement des aimants entre eux garantissant le positionnement et l'orientation uniques de la pièce d'assemblage dans la zone de réception.

Dans un mode de réalisation avantageux pouvant être combiné avec le ou les autres modes de réalisation ci-dessus, le dispositif selon la présente invention comprend en outre dans le fond du chausson un couvercle recouvrant la zone de réception, la face supérieure du couvercle comprenant une cuvette anatomique dimensionnée pour recevoir une face inférieure du moignon.

De préférence, le couvercle est configuré pour se fixer dans le fond du chausson afin de bloquer en translation la pièce d'assemblage dans la zone de réception.

De préférence, le couvercle et la zone de réception sont sensiblement de forme circulaire et comprennent respectivement sur leur pourtour externe et interne un filet et un taraudage aptes à coopérer ensemble pour une fixation par vissage.

De préférence, le couvercle est percé en son centre.

Dans un mode de réalisation avantageux qui peut être combiné avec au moins l'un quelconque des précédents modes, le dispositif selon la présente invention peut comporter en outre un élément de jonction prothétique configuré pour fixer un élément prothétique au corps principal.

De préférence, un tel élément de jonction comporte une platine de fixation assemblée selon une face inférieure externe du corps principal.

Avantageusement, la face inférieure du corps principal comprend des perforations préformées présentant chacune un taraudage pour une insertion et un vissage d'une tige filetée dans chacune des perforations afin de permettre un assemblage de la platine de fixation avec le corps principal.

De préférence, des ouvertures de passage pour chacune des tiges filetées sont ménagées sur la Optionnellement, la platine est percée en son centre.

L'objet de la présente invention concerne selon un deuxième aspect un procédé de fabrication d'un dispositif d'emboîture pour prothèse d'un membre inférieur ou supérieur d'un patient amputé, ledit procédé mis en œuvre par des moyens informatique comprenant les étapes suivantes :
- une étape d'acquisition au cours de laquelle un praticien scanne, par exemple à l'aide d'un outil d'acquisition numérique de type scanner 3D, un moignon d'un membre inférieur ou supérieur d'un patient amputé afin d'obtenir une représentation tridimensionnelle dudit moignon ;
- une étape de génération au cours de laquelle un modèle numérique d'une emboîture est généré à partir de la représentation tridimensionnelle dudit moignon, ledit modèle numérique comprenant une enveloppe surfacique représentative d'un corps principal creux ouvert en partie supérieure et comprenant une paroi latérale et un fond formant un chausson dimensionné pour recevoir au moins partiellement ledit moignon ;
- une étape de détermination d'une position d'une zone de réception se présentant sous la forme d'une empreinte préformée dans le corps principal pour recevoir des moyens d'assujettissement dudit dispositif d'emboîture avec le moignon ;
- une étape de mise à jour dudit modèle par intégration de ladite zone de réception dans le corps principal ;
- une étape d'impression 3D au cours de laquelle, en fonction dudit modèle à jour, on réalise par impression 3D ledit corps principal comprenant ladite empreinte préformée. Avantageusement, la zone de réception est positionnée dans le fond du chausson. Avantageusement, la zone de réception est déterminée en fonction d'un axe déterminé dudit moignon.

Avantageusement, le procédé selon la présente invention comprend une étape de traitement d'images de la représentation tridimensionnelle dudit moignon pour détecter l'axe dudit moignon. Différents algorithmes de traitement d'images peuvent ici être mis en œuvre pour réaliser cette détection.

Dans un mode de réalisation avantageux, la forme de l'empreinte est déterminée en fonction de la forme des moyens d'assujettissement sélectionnés à partir d'une base de données prédéterminée, ladite base de données comprenant une pluralité de moyens d'assujettissement et les formes associées.

De préférence, le procédé selon la présente invention comprend une étape d'enrichissement de la base de données avec notamment des moyens d'assujettissement additionnels ou d'autres pièces ou accessoires.

Dans un mode de réalisation, on prévoit que les moyens d'assujettissement comportent une pièce d'assemblage destinée à être logée dans la zone de réception ; dans ce mode, l'empreinte présente une forme complémentaire à la pièce d'assemblage de manière à ce que la pièce d'assemblage vient s'emboîter dans la zone de réception.

Dans un mode de réalisation qui peut être combiné avec le précédent mode, on prévoit que les moyens d'assujettissement sont actionnables manuellement par actionnement d'un actionneur ; dans ce mode, le procédé selon la présente invention comprend en outre une détermination et un positionnement en fonction dudit actionneur d'une gouttière préformée. De préférence, cette gouttière préformée traverse la paroi latérale en partie inférieure du corps principal pour déboucher dans la zone de réception, la gouttière étant dimensionnée en fonction dudit actionneur de manière à recevoir une portion proximale dudit actionneur et à ce qu'une portion distale de l'actionneur reste accessible au patient lorsque le moignon est en position dans le chausson.

On peut prévoir ici l'utilisation d'une autre base de données (ou la même que celle utilisée précédemment) qui comprend une pluralité d'actionneurs et les formes associées.

Le praticien peut donc lors de cette étape, en sélectionnant l'actionneur souhaité, déterminer dans la base de données la gouttière adaptée ainsi que sa position.

Avantageusement, la pièce d'assemblage comporte un canal radial dont une extrémité débouche sur la paroi latérale externe de la pièce d'assemblage et dont l'autre extrémité débouche sur un canal axial traversant axialement la pièce d'assemblage.

De préférence, le procédé selon la présente invention comprend en outre une détermination et un positionnement d'un détrompeur entre la zone de réception et la pièce d'assemblage pour garantir un positionnement et une orientation unique de la pièce d'assemblage lorsque celle-ci est positionnée dans la zone de réception de manière à aligner la gouttière et le canal radial tout en bloquant en rotation la pièce d'assemblage dans la zone de réception.

De préférence, le détrompeur est déterminé de manière à comporter :
- une empreinte mécanique femelle ou mâle ménagée dans la zone de réception, et
- une empreinte mécanique mâle ou femelle ménagée dans ladite pièce d'assemblage, lesdites empreintes mécaniques mâle et femelle présentant chacune une géométrie complémentaire l'une de l'autre.

Dans un mode de réalisation pouvant être combiné avec les précédents modes, on peut prévoir que les moyens d'assujettissement sont configurés pour assujettir le dispositif d'emboîture au moignon par un assujettissement mécanique d'un plongeur fixé sur un manchon enveloppant le moignon, ledit plongeur se présentant sous la forme d'une tige rigide prolongeant axialement ledit moignon lorsque ledit manchon enveloppe le moignon ; dans ce mode, le procédé selon la présente invention comprend en outre une détermination et un positionnement d'un orifice de passage dans le fond du chausson en fonction de l'axe du moignon de manière à ce qu'une portion du plongeur soit apte à traverser le canal axial et l'orifice de passage pour déboucher à l'extérieur du corps principal.

De préférence, le procédé selon la présente invention comprend en outre une détermination et un positionnement d'un couvercle recouvrant la zone de réception dans le fond du chausson, la face supérieure dudit couvercle comprenant une cuvette anatomique dimensionnée pour recevoir une face inférieure du moignon.

De préférence, le couvercle est percé en son centre pour être aligné avec l'axe du moignon. Avantageusement, le procédé selon la présente invention comprend en outre une détermination et un positionnement de perforations préformées sur la face inférieure du corps principal, chaque perforation présentant de préférence un taraudage pour une insertion et un vissage d'une tige filetée afin de permettre un assemblage d'une platine de fixation avec le corps principal.

Avantageusement, la position des perforations est déterminée en fonction d'un axe d'alignement entre ledit dispositif d'emboîture et des éléments prothétiques sous-jacents au dispositif d'emboîture, ledit axe d'alignement étant défini par une intersection de deux plans, l'un sagittal et l'autre frontal par rapport au moignon.

De préférence, les deux plans sont perpendiculaires entre eux.

Avantageusement, le corps principal comprend une surface interne destinée à être en contact direct avec le moignon et une surface externe, l'épaisseur du corps principal entre la surface interne et la surface externe étant calculée en fonction de données morphologiques du patient dont le poids et/ou de données propres du patient dont le profil pathologique et/ou la dynamique du patient.

De préférence, l'épaisseur est variable.

Avantageusement, lors de l'étape d'impression 3D, le corps principal se présente sous la forme d'une pièce monobloc.

L'objet de la présente invention concerne selon un troisième aspect une utilisation d'un dispositif d'emboîture obtenu par la mise en œuvre d'un procédé de fabrication tel que décrit ci-dessus pour une prothèse d'un membre inférieur d'un patient amputé de type prothèse fémorale ou tibiale.

L'objet de la présente invention concerne selon un quatrième aspect une utilisation d'un dispositif d'emboîture obtenu par la mise en œuvre d'un procédé de fabrication tel que décrit ci-dessus pour une prothèse d'un membre supérieur d'un patient amputé.

L'objet de la présente invention concerne selon un cinquième aspect un appareillage orthopédique pour membre inférieur ou supérieur comprenant au moins un élément prothétique et un dispositif d'emboîture obtenu par la mise en œuvre d'un procédé de fabrication tel que décrit ci-dessus.

Avantageusement, l'au moins un élément prothétique comporte un tibia prothétique et/ou un pied prothétique dans le cadre d'une prothèse fémorale ou tibiale.

Ainsi, par ses différentes caractéristiques techniques fonctionnelles et structurelles décrites ci-dessus, l'orthoprothésiste dispose d'un process de fabrication permettant d'offrir au patient amputé une solution simple et efficace pour fournir un dispositif d'emboîture confortable, de qualité et adaptée à la morphologie du moignon du patient, ceci en un minimum de temps.

### Brève description des figures annexées

D'autres caractéristiques et avantages de la présente invention ressortiront de la description ci-dessous, en référence aux figures annexées qui en illustrent un exemple de réalisation dépourvu de tout caractère limitatif et sur lesquelles :
[Fig.1]

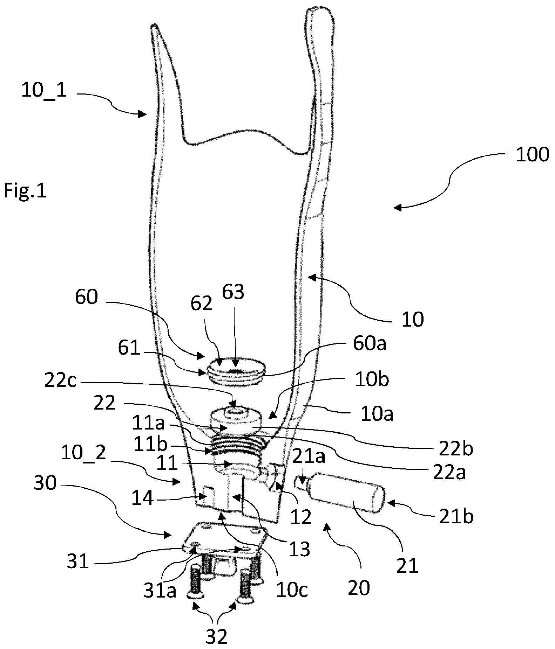

   La figure 1 représente une vue éclatée d'une coupe transversale en perspective d'un dispositif d'emboîture selon un exemple de réalisation de la présente invention ;
[Fig.2]

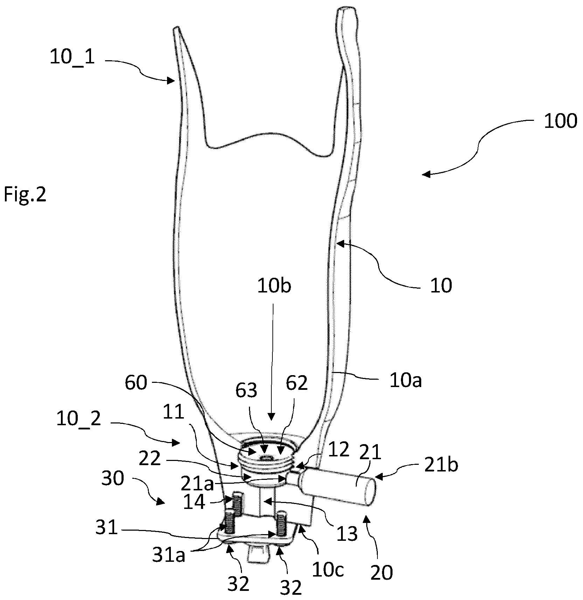

   La figure 2 représente une coupe transversale en perspective d'un dispositif d'emboîture conforme à l'exemple de la figure 1 ;
[Fig.3]

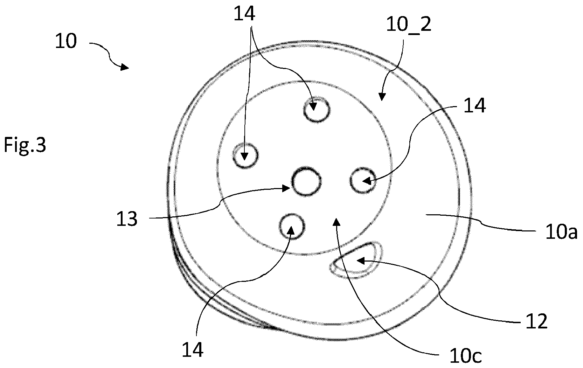

   La figure 3 représente une vue de dessous d'un dispositif d'emboîture selon un exemple de réalisation de la présente invention ;
[Fig.4]

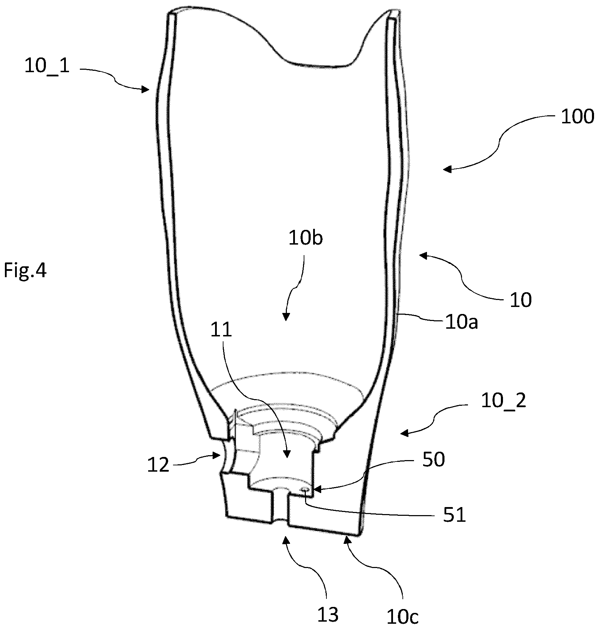

   La figure 4 représente une autre coupe transversale en perspective d'un dispositif d'emboîture selon un exemple de réalisation de la présente invention mettant en évidence un détrompeur pour la pièce d'assemblage dans le fond du chausson ;
[Fig.5]

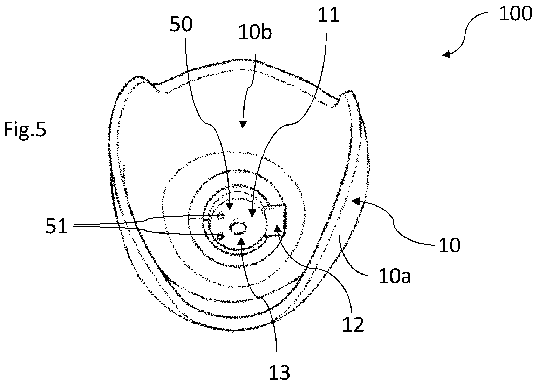

   La figure 5 représente une vue de dessus d'un dispositif d'emboîture selon un exemple de réalisation de la présente invention mettant en évidence un détrompeur pour la pièce d'assemblage dans le fond du chausson ;
[Fig.6]

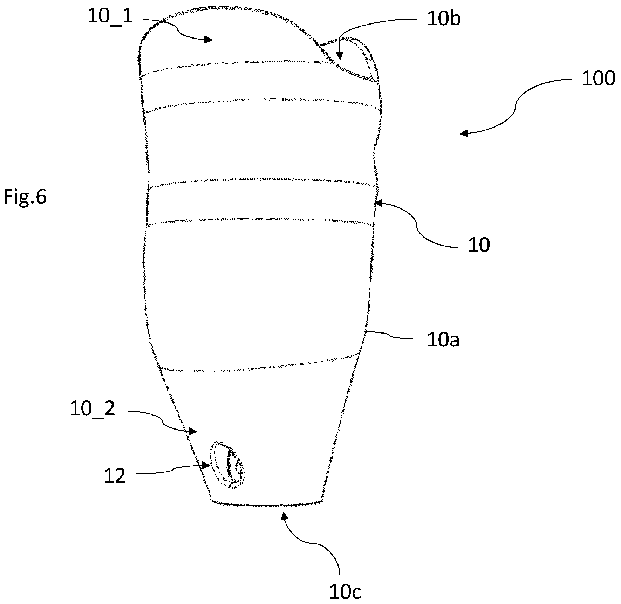

   La figure 6 représente une vue en perspective d'un dispositif d'emboîture selon un exemple de réalisation de la présente invention mettant en évidence un canal radial ménagé dans la paroi latérale du corps principal ; et
[Fig.7]

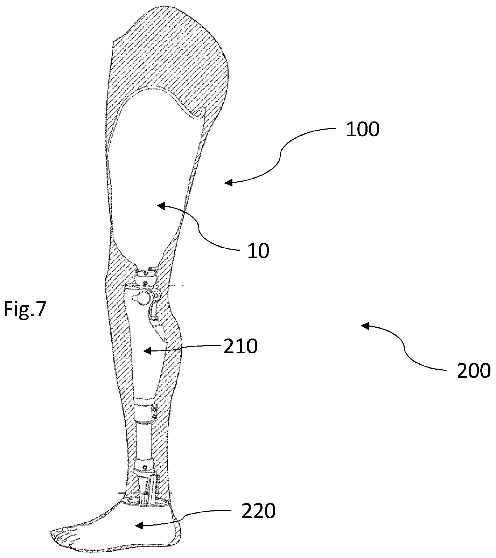

   La figure 7 représente une vue schématique d'un appareillage prothétique comprenant un dispositif d'emboîture selon un exemple de réalisation de la présente invention ;
[Fig.8]

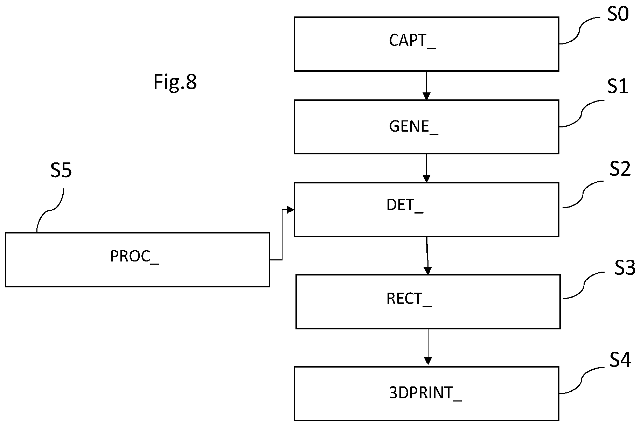

   La figure 8 représente un organigramme représentant les principales étapes du procédé de fabrication selon un exemple de réalisation de la présente invention ;
[Fig.9]

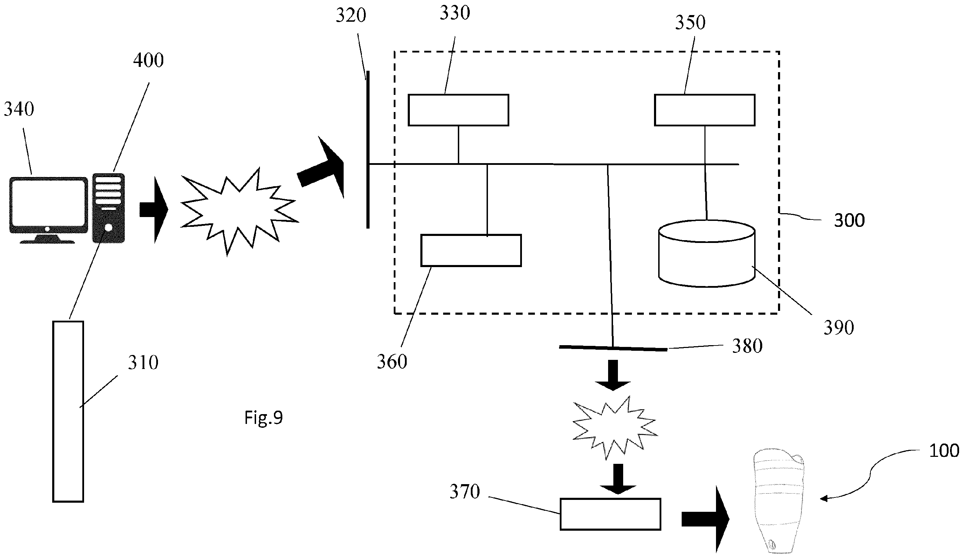

   La figure 9 représente une vue schématique d'un système pour la fabrication d'un dispositif d'emboîture selon un exemple de réalisation de la présente invention ; et
[Fig.10]

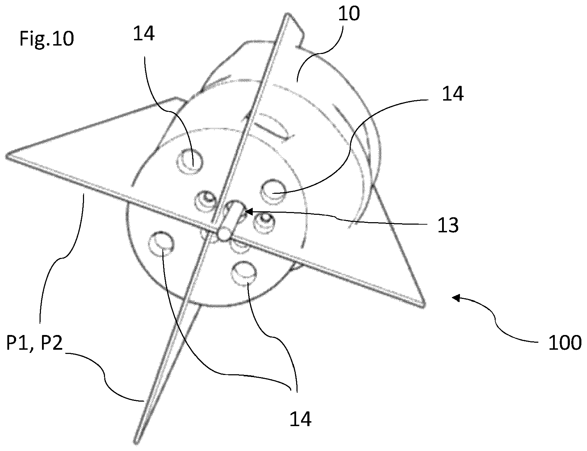

   La figure 10 représente une modélisation 3D d'un dispositif d'emboîture selon un exemple de réalisation de la présente invention.

### Description détaillée selon un exemple de réalisation avantageux

Un dispositif d'emboîture pour prothèse fémorale selon un exemple de réalisation de la présente invention ainsi que le procédé de fabrication qui lui est associé vont maintenant être décrits dans ce qui va suivre en référence conjointement aux figures 1 à 10.

Pour rappel, un des objectifs de la présente invention consiste à fournir à un patient amputé d'un membre inférieur ou supérieur un appareillage prothétique confortable à porter dont le processus de fabrication n'est pas contraignant pour le patient.

Un des autres objectifs de la présente invention est de proposer une technique de fabrication qui soit fiable et simple à mettre en œuvre pour l'orthoprothésiste tout en limitant les produits non-recyclables et les étapes nécessitant un outillage spécifique.

Encore un des autres objectifs de la présente invention est de proposer une technique de fabrication d'un dispositif d'emboîture permettant d'adapter tous les types de système d'accroché.

Ces différents objectifs sont atteints avec la fourniture d'une technologie de modélisation et d'impression 3D permettant la fabrication via un outil logiciel d'un dispositif d'emboîture comprenant des préformages pour assembler ladite emboîture 100 avec les éléments prothétiques 210 et 220 permettant la fabrication définitive d'un appareillage prothétique 200 tel que décrit ci-après et illustré en figure 7.

Dans l'exemple décrit ici, l'appareillage 200 est destiné à un patient amputé du fémur ; il s'agit donc d'un exemple portant sur une prothèse fémorale, une telle prothèse comprenant un dispositif d'emboîture 100 relié à un tibia prothétique 210 connecté à un pied prothétique 220 comme illustré en figure 7.

Il s'agit d'un exemple de mise en œuvre parmi d'autres exemples possibles. L'homme du métier comprendra en effet que le concept inventif décrit ici pour un appareillage prothétique 200 de type prothèse fémorale peut également s'adapter aux prothèses dites tibiales mais également aux prothèses des membres supérieurs.

Le dispositif d'emboîture 100 d'un tel appareillage 200 présente une conception monobloc réalisée à partir d'une pièce unique obtenue par impression 3D.

L'obtention d'une telle pièce monobloc par impression 3D est caractéristique de la présente invention.

Dans l'exemple décrit ici et comme illustré en figure 8, l'orthoprothésiste lors d'un premier rendez-vous avec le patient amputé réalise lors d'une étape d'acquisition S0 un scan du moignon du patient à l'aide d'un outil 310 de type scanner 3D.

Cette étape S0 préalable au process permet l'obtention d'une représentation tridimensionnelle du moignon avec la génération d'un fichier informatique comprenant une telle représentation du moignon.

Dans cet exemple, on comprend qu'à l'issue de cette étape S0, l'orthoprothésiste détient grâce au scan 3D une version numérique d'un positif du membre résiduel du patient (le moignon) enregistré dans un fichier informatique au format STL (fichier 3D).

Grâce à la mise en œuvre d'une technologie de modélisation numérique du moignon, aucun coulage n'est nécessaire : cette étape d'acquisition numérique S0 d'un membre d'un patient amputé est réalisée dans un bureau à l'aide d'un simple ordinateur 400 sur lequel est installé en local un logiciel dédié et un outil 310 de type scanner 3D.

Ce logiciel dédié peut également être en version « *cloud* » en étant accessible à distance sur un portail via un serveur distant (voir figure).

Dans l'exemple décrit ici et illustré en figures 9 et 10, le fichier généré est envoyé au système 300.

On prévoit donc ici une interface de communication 320 apte à recevoir du serveur le fichier STL. Après réception, le logiciel métier mis en œuvre sur le système 300 et développé dans le cadre de la présente invention génère lors d'une étape SI à partir dudit fichier STL reçu (et donc de ladite représentation tridimensionnelle dudit moignon) un modèle numérique représentatif d'une enveloppe surfacique apte à envelopper au moins partiellement le moignon du patient.

Une telle génération SI dudit modèle est réalisée par un circuit de génération 330 tel qu'un processeur qui détermine le modèle de l'emboîture 100 en tant que négatif du moignon.

Le premier modèle généré se présente donc comme une enveloppe comprenant un corps principal 10 creux qui est ouvert en partie supérieure 10_1 pour permettre l'insertion du moignon et qui comprend une paroi latérale 10b et un fond 10b formant ensemble un chausson dimensionné pour recevoir au moins partiellement le moignon.

A partir d'une interface graphique 340 de type GUI et du logiciel dédié, le praticien travaille lors d'une étape S2 le modèle généré de manière à concevoir un modèle d'emboîture définitif pouvant être porté par le patient.

Celui-ci détermine une épaisseur du corps principal 10. Le corps 10 comprend une surface interne destinée à être en contact direct avec le moignon et une surface externe, l'épaisseur du corps principal 10 étant définie par la distance de séparation entre la surface interne et la surface externe.

Cette épaisseur est variable et peut être ajustée par le praticien pour assurer le confort du patient.

Ces différentes épaisseurs du corps principal 10 peuvent donc être déterminées lors de l'étape S2 par le praticien. Dans cet exemple, l'épaisseur de chaque zone du corps principal 10 peut également être calculée par le logiciel en fonction de données morphologiques dudit patient dont le poids et/ou de données propres du patient dont le profil pathologique et/ou la dynamique du patient.

Un calculateur (non représenté ici) ou le processeur 330 propose alors des valeurs d'épaisseurs possibles pour chaque zone du corps 10.

Cette étape de détermination S2 sera décrite plus en détails ci-après.

Dans l'exemple décrit ici et comme illustré en figures 4 et 6, le corps principal 10 conçu numériquement lors de l'étape SI comprend donc une paroi latérale 10b et un fond 10b formant un chausson dimensionné pour recevoir au moins partiellement un moignon d'un membre inférieur d'un patient amputé (ici le fémur).

Dans le produit fini, on prévoit la mise en œuvre de moyens d'assujettissement 20, de tels moyens 20 étant configurés pour assurer un assujettissement du dispositif d'emboîture 100 avec le moignon.

A partir d'une interface graphique 340 de type GUI et du logiciel dédié, le praticien détermine donc lors de cette étape S2 une position d'une zone de réception 11 de ces moyens d'assujettissement 20.

Cette position est fonction de l'axe du moignon.

On peut prévoir une étape de traitement d'images S5 sur la représentation tridimensionnelle dudit moignon pour détecter cet axe. Différents algorithmes de traitement d'images peuvent ici être mis en œuvre pour réaliser une détection précise et rapide de l'axe.

En fonction des moyens sélectionnés 20 par le praticien via une base de données 390, ces moyens peuvent présenter une forme spécifique qu'il faut pouvoir positionner convenablement dans le fond du corps principal 10.

Dans cet exemple, la zone de réception 11 se présente sous la forme d'une empreinte préformée dans le corps principal 10 pour recevoir des moyens d'assujettissement 20 Comme illustré en figure 4 et comme expliqué précédemment, l'empreinte préformée est donc dimensionnée pour recevoir une pièce d'assemblage 22.

La base de données 390 comprend donc des données contenant des informations relatives aux différentes pièces d'assemblage possibles ainsi que les formes associées.

Toutes les formes et les dimensions des pièces d'assemblages 22 (ainsi que les autres pièces et accessoires décrits ci-après) sont ainsi stockées dans cette base de données 390 qui peut être alimentée au fil du temps. Ainsi, les différentes pièces d'assujettissement disponibles sur le marché sont modélisées en 3D et peuvent être ajoutées progressivement pour enrichir et personnaliser cette base de données 390 proposée aux orthoprothésistes, ceci en fonction des besoins et des préférences du patricien.

Le système 300 est donc capable de déterminer une forme d'empreinte en fonction de la pièce d'assemblage 22 sélectionnée préalablement dans la base de données 390.

Grâce à l'utilisation du logiciel, la pièce d'assemblage 22 et la zone de réception 11 sont donc configurées et dimensionnés l'une par rapport à l'autre de manière à permettre un positionnement bord contre bord de la pièce 22 dans la zone de réception 11.

On comprend que la zone de réception 11 présente une forme complémentaire à celle de la pièce 22 de manière à ce que ladite pièce 22 vienne s'emboîter dans la zone 11.

Dans l'exemple décrit ici et comme illustré en figure 1, cette pièce d'assemblage 22 se présente sous la forme d'une pièce sensiblement cylindrique avec un épaulement 22a permettant un appui de la pièce 22 contre le fond de la zone de réception 11. Alternativement, on peut prévoir d'autres formes telles que par exemple un biseautage de la partie inférieure de la pièce d'assemblage 22.

Dans cet exemple, la pièce d'assemblage 22 illustrée notamment en figure 1 vient en liaison avec un actionneur 21 lorsque lorsqu'on positionne cette pièce 22 dans la zone de réception 11.

L'actionneur 21 doit être accessible en paroi latérale 10a du chausson tandis que la pièce d'assemblage 22 est logée à l'intérieur du volume défini par le chausson, dans le fond 10b de celui-ci.

La mise en liaison de l'actionneur 21 et de la pièce d'assemblage 22 est rendue possible par la détermination et le positionnement d'une gouttière 12 préformée en fonction de l'actionneur 21 sélectionné et qui est capable de traverser la paroi latérale 10a en partie inférieure 10_2 pour déboucher dans la zone de réception 11.

Le praticien peut donc lors de l'étape S2 sélectionner via le logiciel et une base de données 390 dédiée l'actionneur 21 souhaité.

Le processeur 330 détermine ensuite en fonction des informations relatives à l'encombrement de cette pièce 21 les dimensions de la gouttière 12.

Lors de l'impression 3D, cette gouttière 12 sera alors dimensionnée pour recevoir une portion proximale 21a de l'actionneur 21. On peut par exemple prévoir que cette portion proximale 21a vient se visser dans la gouttière 12.

La portion distale 21b de l'actionneur 21 sera alors accessible par le patient, l'actionnement de l'actionneur 21 par la portion distale 21b permettant un assujettissement du dispositif 100 avec le moignon du patient. C'est cette portion 21b qui comprend les moyens permettant l'actionnement de l'actionneur 21 (par exemple une molette ou un bouton poussoir). L'utilisation du logiciel permet d'assister le praticien dans la conception du modèle numérique en proposant un préformage de cette gouttière 12 dans le corps principal 10.

Dans l'exemple décrit ici, cette pièce d'assemblage 22 comprend un canal radial (non représenté ici) dont une extrémité débouche sur la paroi latérale externe 22b de la pièce 22 et dont l'autre extrémité débouche sur un canal axial 22c traversant axialement la pièce 22. Dans l'exemple décrit ici, il est souhaitable de prévoir un alignement de la gouttière 12 avec le canal radial.

Dans cet exemple, on prévoit ainsi la mise en œuvre d'un détrompeur 50 entre la zone de réception 11 et la pièce d'assemblage 22, un tel détrompeur 50 garantissant un positionnement et une orientation unique de la pièce d'assemblage 22 lorsque celle-ci est positionnée dans la zone de réception 11. On comprend plus particulièrement que ce détrompeur 50 a pour fonction d'aligner ensemble la gouttière 12 et le canal radial tout en bloquant en rotation la pièce d'assemblage 22 dans la zone de réception 11.

Un tel alignement permet de connecter entre eux l'actionneur 21 et la pièce d'assemblage 22 pour assujettir le moignon au dispositif d'emboîture 100.

Il est ainsi possible au praticien lors de l'étape S2 de sélectionner le détrompeur 50 en fonction de la pièce d'assemblage 22 sélectionnée.

Dans l'exemple décrit ici et comme illustré en figures 4 et 5, le détrompeur 50 comporte une empreinte mécanique femelle 51 ménagée dans la zone de réception 11 et une empreinte mécanique mâle ménagée sous la pièce d'assemblage 22.

Dans l'exemple décrit ici, ces empreintes mécaniques mâle et femelle 51 sont désaxées et présentent chacune une géométrie complémentaire l'une de l'autre de manière à ce que, lorsque on fait coopérer ces empreintes ensemble en positionnant la pièce 22 dans la zone 11, on est contraint de positionner et d'orienter la pièce d'assemblage 22 selon une orientation et un positionnement unique dans la zone de réception 11, cette orientation et ce positionnement uniques assurant un alignement du canal radial et de la gouttière 12.

La pièce d'assemblage 22 étant bloquée en rotation dans la zone de réception 11 grâce au détrompeur 50, on prévoit ensuite le positionnement d'un couvercle anatomique 60 qui vient se visser par exemple dans le fond 10b du chausson afin de bloquer en translation ladite pièce d'assemblage 22 dans la zone de réception 11 ; un tel vissage étant possible grâce à la mise en œuvre d'un couvercle 60 et d'une zone de réception chacun de forme circulaire avec la présence d'un filet 61 et d'un taraudage 11b respectivement sur le pourtour interne 11a de la zone de réception et sur le pourtour externe 60a du couvercle 60.

Dans l'exemple décrit ici, le couvercle 60 présente une face supérieure formant une cuvette 62 dimensionnée pour recevoir la face inférieure du moignon.

Comme illustré en figure 1, le couvercle 60 est percé axialement en son centre et présente donc un trou 63 aligné avec un orifice de passage 13 ménagé dans le fond 10b du chausson et le canal axial 22c de la pièce d'assemblage 22.

Dans cet exemple, les moyens d'assujettissement 20 sont du type accroche distale.

De tels moyens d'assujettissement 20 impliquent la mise en œuvre d'un plongeur (non représenté ici) se présentant sous la forme d'une tige rigide prolongeant axialement le moignon lorsque celui-ci est enveloppé par un manchon, ledit plongeur étant solidaire du manchon.

On comprend donc que, lorsqu'un patient portant un tel manchon couplé à un plongeur enfile l'emboîture 100, le plongeur est orienté axialement vers le fond 10b du chausson et traverse successivement le trou 63, le canal axial 22c et l'orifice de passage 13.

Pour assujettir le dispositif 100 au moignon, on prévoit dans cet exemple la mise en œuvre d'un système de verrouillage/déverrouillage (non représenté ici) logé dans le canal radial.

Par exemple, un tel système de verrouillage/déverrouillage comprend une pièce de verrouillage/déverrouillage mobile montée sur ressort et apte à présenter deux positions dont une position de déverrouillage dans laquelle la pièce reste dans le volume défini par le canal radial et une position de verrouillage dans laquelle une portion de la pièce s'engage dans le volume défini par le canal axial de manière à venir en butée contre le plongeur pour maintenir en position celui-ci dans le canal axial.

On comprend donc que la pièce de verrouillage/déverrouillage agit comme un verrou qui vient compresser le plongeur dans le canal axial de manière à bloquer en translation celui-ci. L'actionnement de ce verrou est mis en œuvre par la portion distale 21b de l'actionneur 21 que l'on peut manipuler de l'extérieur de l'emboîture 100, par exemple par une molette ou un bouton poussoir.

On peut prévoir d'autres moyens d'assujettissement 20 comme par exemple des moyens d'assujettissement par dépressurisation.

Dans cette alternative qui peut aussi être combinée avec un assujettissement mécanique avec plongeur, l'assujettissement (complémentaire ou non) est assuré par une dépressurisation du volume entre le moignon et l'emboîture.

Il est donc prévu soit une deuxième gouttière (non représentée ici) ménagée dans la paroi latérale 10a du corps principal 10 pour l'introduction de ces moyens par dépressurisation (cas dans lequel on prévoit les deux systèmes d'assujettissement), soit l'utilisation de la gouttière 12 pour l'introduction des moyens par dépressurisation (cas dans lequel les moyens d'assujettissement par dépressurisation assurent à eux seuls l'assujettissement).

Dans l'exemple décrit ici, on prévoit également la mise en œuvre d'un élément de jonction prothétique 30 assurant la jonction entre l'emboîture 100 et le tibia prothétique 210.

Ici, l'élément de jonction 30 comprend une platine de fixation 31 venant se fixer sous la face inférieure 10c du corps principal 10 par un système de visserie 32 traversant la platine 31 grâce à des orifices de passage 31a alignés avec des perforations 14 ménagées sous la face inférieure 10c du corps principal.

Dans cet exemple, la position des perforations 14 est déterminée par un axe d'alignement formé par l'intersection de deux plans P1 et P2, cet axe définissant l'alignement de l'emboîture 100 par rapport aux pièces prothétiques sous-jacentes à emboîtures qui constituent la prothèse.

Dans cet exemple, les deux plans P1 et P2, l'un frontal et l'autre sagittal, sont perpendiculaires l'un à l'autre.

Cet alignement est donc déterminé par le logiciel de 3D.

Pour ce faire, l'orthoprothésiste réalise une translation de ces plans P1 et P2, dans une vue frontale pour le plan sagittal et dans une vue sagittale pour le plan frontal.

Une fois l'axe d'alignement déterminé, il est possible de positionner correctement ces perforations 14 sous la face inférieur 10c du corps principal 10 pour fixer la platine 31 par rapport à cet axe d'alignement.

On comprend donc ici que tout l'intérêt de la conception de la modélisation 3D de l'emboîture 100 : Ceci offre notamment la possibilité au praticien de prévoir dans la pièce elle-même les formes et les positions précises et correctes des ouvertures traversantes 12 et 13 préformées débouchant chacune dans la zone de réception 11 au fond du chausson de l'emboîture 100 ainsi que des perforations 14 préformées pour la fixation de la platine 31 sous le corps principal 10.

Suite à cette étape S2, on prévoit une mise à jour S3 du modèle initialement généré de manière à ce que le fichier intègre l'ensemble des données relatives aux formes et aux positions des zones 11, des ouvertures 12 et 13 ainsi que des perforations 14.

Cette mise à jour S3 est réalisée par l'intermédiaire d'un circuit 350.

Ceci permet de générer un nouveau fichier STL correspondant à la forme de l'emboîture 100 souhaitée avec l'ensemble des éléments nécessaires à la fabrication et l'assemblage de l'emboîture 100 avec les éléments prothétiques sous-jacents 210 et 220.

Lors de cette étape S3, on enregistre donc l'ensemble de ces nouvelles données sur des moyens de mémorisation 360, voire d'alimenter un algorithme de *learning machine* pour améliorer les performances du logiciel de conception.

On génère donc après cette étape S3 un nouveau fichier à jour avec l'ensemble des informations pertinentes pour la réalisation d'un dispositif d'emboîture 100 sur mesure pour le patient.

Une fois ce fichier généré, il est possible de l'envoyer vers une imprimante 3D, notée ici 370, via par exemple une deuxième interface de communication sans fil 380.

Le process prévoit alors la réalisation d'une impression 3D, notée S4, du corps principal 10 du dispositif 100 avec la zone de réception 11 dédiée au positionnement des moyens d'assujettissement 20 et l'ensembles des autres zones 11, des ouvertures 12 et 13 ainsi que des perforations 14.

L'utilisation de l'informatique et de l'impression 3D permet une modélisation de l'emboîture et une construction numérique d'un modèle avant impression 3D, ce qui permet de faire disparaître définitivement l'utilisation du plâtre, ce qui améliore le confort du patient et réduit le temps de travail de l'orthoprothésiste qui peut se concentrer sur son travail de base et son expertise.

L'utilisation de l'informatique et de l'impression 3D a également pour avantage de supprimer les temps de nettoyage liés à la matière première.

La réalisation d'un tel corps principal 10 par impression 3D est caractéristique de la présente invention.

L'étape de détermination S2 présente de nombreuses fonctionnalités facilitant le travail du praticien.

La séquence 3D mise au point par le Demandeur dans le cadre de la présente invention est une combinaison des technologies existantes en permettant un scan 3D du moignon, une rectification 3D, une modélisation 3D de l'emboîture et une impression 3D de celle-ci.

La combinaison de ces différentes technologies au sein d'un processus réfléchi élimine une multitude d'inconvénients présent dans la séquence de référence ; la présente invention fait par ailleurs évoluer la mise en pratique du savoir-faire de l'orthoprothésiste en permettant une application immédiate de l'enseignement théorique reçu.

La séquence ainsi mise au point permet non seulement de réaliser des emboîtures tibiales et fémorales provisoires, mais aussi des emboîtures pour membre supérieur.

Avec le développement d'un processus supplémentaire pour l'acquisition du fichier STL initial, le même processus global permet de réaliser une multitude d'autres appareils prothétiques sur mesure : prothèses (remplace un membre) et orthèses (pallies le dysfonctionnement d'un membre).

Le pré-formatage des emboîtures est également un avantage significatif de la présente invention, permettant ainsi aux praticiens d'avoir une solution clé en main.

L'emboîture proposée dans le cadre de la présente invention peut ainsi être fabriquée et livrée prête à monter : aucune manipulation supplémentaire n'est nécessaire pour mettre en place les pièces prothétiques sous-jacentes.

Il devra être observé que cette description détaillée porte sur plusieurs exemples de réalisation particuliers de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Procédé de fabrication d'un dispositif d'emboîture (100) pour prothèse d'un membre inférieur ou supérieur d'un patient amputé, ledit procédé mis en œuvre par des moyens informatique comprenant les étapes suivantes :
- une acquisition (S0) d'une représentation tridimensionnelle d'un moignon du membre inférieur ou supérieur dudit patient amputé ;
- une génération (S1) d'un modèle numérique d'un dispositif d'emboîture (100) à partir de ladite représentation tridimensionnelle dudit moignon, ledit modèle numérique comprenant une enveloppe surfacique représentative d'un corps principal (10) creux ouvert en partie supérieure (10_1) et comprenant une paroi latérale (10b) et un fond (10b) formant un chausson dimensionné pour recevoir au moins partiellement ledit moignon ;
- une détermination (S2) d'une position d'une zone de réception (11) se présentant sous la forme d'une empreinte préformée dans le corps principal (10) pour recevoir des moyens d'assujettissement (20) dudit dispositif d'emboîture (100) avec le moignon ;
- une mise à jour (S3) dudit modèle par intégration de ladite zone de réception (11) dans ledit corps principal (10) ;
- une impression 3D (S4) dudit corps principal (10) comprenant ladite empreinte préformée en fonction dudit modèle à jour.

2. Procédé selon la revendication 1, dans lequel la zone de réception (11) est positionnée dans le fond (10b) dudit chausson.

3. Procédé selon la revendication 1 ou 2, dans lequel la zone de réception (11) est déterminée en fonction d'un axe prédéterminé dudit moignon.

4. Procédé selon la revendication 3, lequel comprend un traitement d'images (S5) de la représentation tridimensionnelle dudit moignon pour détecter ledit axe dudit moignon.

5. Procédé selon l'une quelconque des revendications précédentes, dans lequel la forme de l'empreinte est déterminée en fonction de la forme des moyens d'assujettissement (20) sélectionnés à partir d'une base de données (390) prédéterminée, ladite base de données (390) comprenant une pluralité de moyens d'assujettissement (20) et les formes associées.

6. Procédé selon la revendication 5, les moyens d'assujettissement (20) comportant une pièce d'assemblage (22) destinée à être logée dans la zone de réception (11),
dans lequel l'empreinte préformée présente une forme complémentaire à ladite pièce d'assemblage (22) de manière à ce que ladite pièce d'assemblage (22) vient s'emboîter dans ladite zone de réception (11).

7. Procédé selon l'une quelconque des revendications précédentes, lesdits moyens d'assujettissement (20) étant actionnables manuellement par actionnement d'un actionneur (21),
dans lequel l'étape de détermination (S2) comprend une détermination et un positionnement en fonction dudit actionneur (21) d'une gouttière (12) préformée traversant ladite paroi latérale (10a) en partie inférieure (10_2) dudit corps principal (10) pour déboucher dans ladite zone de réception (11), ladite gouttière (12) étant dimensionnée de manière à recevoir une portion proximale (21a) dudit actionneur (21) et à ce qu'une portion distale (21b) dudit actionneur (21) reste accessible audit patient lorsque ledit moignon est en position dans ledit chausson.

8. Procédé selon la revendication 7 rattachée à la revendication 6, la pièce d'assemblage (22) comportant un canal radial dont une extrémité débouche sur la paroi latérale externe de la pièce d'assemblage (22) et dont l'autre extrémité débouche sur un canal axial traversant axialement la pièce d'assemblage (22),
dans lequel l'étape de détermination (S2) comprend une détermination et un positionnement d'un détrompeur (50) entre la zone de réception (11) et la pièce d'assemblage (22) pour garantir un positionnement et une orientation unique de la pièce d'assemblage (22) lorsque celle-ci est positionnée dans la zone de réception (11) de manière à aligner ladite gouttière (12) et ledit canal radial tout en bloquant en rotation ladite pièce d'assemblage (22) dans ladite zone de réception (11).

9. Dispositif (100) selon la revendication 8, dans lequel le détrompeur (50) est déterminé de manière à comporter :
- une empreinte mécanique (51) femelle ou mâle ménagée dans la zone de réception (11), et
- une empreinte mécanique mâle ou femelle ménagée dans ladite pièce d'assemblage (22),
lesdites empreintes mécaniques (51) mâle et femelle présentant chacune une géométrie complémentaire l'une de l'autre.

10. Procédé selon la revendication 8 ou 9, les moyens d'assujettissement (20) étant configurés pour assujettir ledit dispositif d'emboîture (100) audit moignon par un assujettissement mécanique d'un plongeur fixé sur un manchon enveloppant ledit moignon, ledit plongeur se présentant sous la forme d'une tige rigide prolongeant axialement ledit moignon lorsque ledit manchon enveloppe ledit moignon,
dans lequel l'étape de détermination (S2) comprend une détermination et un positionnement d'un orifice de passage (13) dans le fond (10b) dudit chausson en fonction de l'axe dudit moignon de manière à ce qu'une portion dudit plongeur soit apte à traverser le canal axial et l'orifice de passage (13) pour déboucher à l'extérieur dudit corps principal (10).

11. Procédé selon l'une quelconque des revendications précédentes, dans lequel l'étape de détermination (S2) comprend une détermination et un positionnement d'un couvercle (60) recouvrant la zone de réception (11) dans le fond (10b) dudit chausson, la face supérieure dudit couvercle (60) comprenant une cuvette anatomique dimensionnée pour recevoir une face inférieure dudit moignon.

12. Procédé selon l'une quelconque des revendications précédentes, dans lequel l'étape de détermination (S2) comprend une détermination et un positionnement de perforations (14) préformées sur la face inférieure (10c) du corps principal (10), chaque perforation présentant un taraudage pour une insertion et un vissage d'une tige filetée (32) afin de permettre un assemblage d'une platine de fixation (31) des éléments prothétiques sous-jacents au dispositif d'emboîture (100) avec le corps principal (10).

13. Procédé selon la revendication 12, dans lequel la position des perforations est déterminée en fonction d'un axe d'alignement entre ledit dispositif d'emboîture (100) et des éléments prothétiques sous-jacents au dispositif d'emboîture (100), ledit axe d'alignement étant défini par une intersection de deux plans (P1, P2), l'un sagittal et l'autre frontal par rapport au moignon, lesdits deux plans (P1, P2) étant de préférence perpendiculaires entre eux.

14. Procédé selon l'une quelconque des revendications précédentes, dans lequel le corps principal (10) comprend une surface interne destinée à être en contact direct avec ledit moignon et une surface externe, l'épaisseur du corps principal (10) entre la surface interne et la surface externe étant calculée en fonction de données morphologiques dudit patient dont le poids et/ou de données propres du patient dont le profil pathologique et/ou la dynamique du patient.

15. Procédé selon la revendication 14, dans lequel l'épaisseur est variable.
